# EUROPÄISCHE PATENTANMELDUNG

(11) **EP 0 623 599 A1**
(43) Veröffentlichungstag der Anmeldung: **09.11.1994**
(21) Anmeldenummer: 94810166.2
(22) Anmeldetag: 17.03.1994
(51) Int. Cl.: C07D 219/08, C07D 311/82, G01N 31/22

(54) **Optischer Sensor zur Bestimmung von Kationen**

(30) Priorität: 26.03.1993 CH 928/93
(71) Anmelder: CIBA-GEIGY AG, CH-4002 Basel (CH)
(72) Erfinder: Alder, Alex, Dr., CH-4422 Arisdorf (CH); Barnard, Steven, Dr., CH-4057 Basel (CH)

(57) **Zusammenfassung**

Verbindungen der Formel I und II 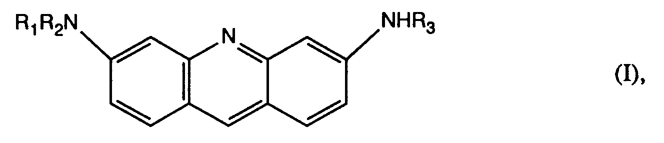 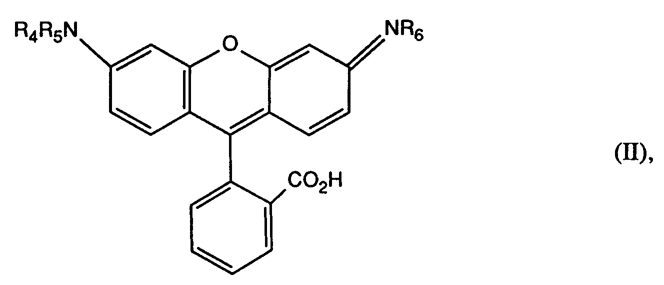
worin R₁ und R₃ sowie R₄ und R₆ für C₁-C₃₀-Alkyl oder C₁-C₃₀-Alkyl-CO- und R₂ sowie R₅ für H oder C₁-C₃₀-Alkyl stehen, mit der Massgabe, dass die Gesamtzahl der C-Atome der Alkylgruppen mindestens 12 beträgt, und deren Salze mit anorganischen oder organischen Säuren.

Diese Verbindungen sind Fluorophore mit hoher Basizität, die sich als Fluorophore in Membranen zur optischen Bestimmung von Kationen in wässrigen Medien, besonders Kaliumionen, durch die Messung insbesondere der konzentrationsabhängigen Erniedrigung der Fluoreszenz eignen.

## Beschreibung

Die Erfindung betrifft einen Sensor zur optischen Bestimmung von Kationen aus der Gruppe von Metall- und Ammoniumkationen (im folgenden als Kationen bezeichnet) in wässrigen Messproben nach der Fluoreszenzmethode, der bestimmte stark basische Farbstoffe aus der Gruppe der Rhodamine und Acridine als Fluorophore in der aktiven Schicht enthält, und ein Verfahren zur qualitativen oder quantitativen Bestimmung von Kationen besonders in wässrigen Lösungen unter Verwendung des optischen Sensors. Die Erfindung betrifft ferner bestimmte Acridine und Rhodamine sowie deren Verwendung als Fluorophore in Sensoren zur optischen Bestimmung von Kationen in wässrigen Proben.

Die optische Bestimmung von Metallkationen hat in letzter Zeit eine erhöhte Bedeutung erlangt, wobei man die Gegenwart oder Konzentration von Metallkationen zum Beispiel über die Veränderung der Absorption oder Fluoreszenz eines geeigneten Farbstoffs misst. Die auch als Optroden bezeichneten Sensoren bestehen im allgemeinen aus einem transparenten Trägermaterial und einer aktiven Schicht. Diese aktive Schicht enthält in der Regel ein transparentes hydrophobes Polymer und einen lipophilen Weichmacher zur Erzielung einer ausreichenden Ionendiffusion und Löslichkeit der aktiven Bestandteile. Als aktive Bestandteile sind ein spezifischer Ionophor als Komplexbildner für Metallkationen, ein lipophiles Salz als Gegenion zur Aufrechterhaltung der elektrischen Neutralität und eine Indikatorsubstanz, die auf Grund einer chemischen Veränderung oder einer physikalischen Veränderung der Umgebung ein messbares optisches Signal ergibt.

In der US-A-4 645 744 sind solche Systeme beschrieben, die als Indikatorsubstanz eine neutrale Verbindung wie zum Beispiel einen Farbstoff (p-Nitrophenol) enthalten, der eine Wechselwirkung mit einem Ionophor/Metallkationenkomplex eingeht, aus der eine Farbänderung als optisch messbares Signal resultiert. Die Wechselwirkung kann beispielsweise die Abspaltung eines Protons aus dem Farbstoff verursachen, wodurch eine Veränderung des Elektronenzustands erzielt wird. Als geeignet werden auch fluoreszierende Verbindungen (zum Beispiel Fluorescein) beschrieben, deren Fluoreszenz durch die Änderung im Elektronenzustand verändert und optisch mittels Fluoreszenzmessungen bestimmt werden kann.

H. He et al. beschreiben in Chemical, Biochemical and Environmental Fiber Sensors II, SPIE Vol. 1368, Seiten 165 bis 174 (1990) Systeme mit einem Protonenträger (Nilblau, Nile Blue)) als Indikatorsubstanz, wobei durch den Transport von Kalium in die aktive Schicht mittels Valinomycin als Ionophor der Protonenträger dissoziiert und ein Proton in die wässrige Phase diffundiert. Der Protonenträger ändert seine Farbe von blau nach rot und je nach Wahl der Wellenlänge kann entweder die Verminderung der Fluoreszenz des blauen Farbstoffs oder die entsprechende Erhöhung der Fluoreszenz des roten Farbstoffs bestimmt werden. Wegen der höheren Empfindlichkeit und Selektivität wird die Messung der Fluoreszenz bevorzugt. Ein wesentlicher Nachteil des Verfahrens ist die geringe Empfindlichkeit des Systems, die auf der geringen Fluoreszenzquantenausbeute des verwendeten Indikatorfarbstoffs beruht.

J. N. Roe beschreibt in Analyst, Vol. 115, Seiten 353 bis 358 (1990) ein System, das auf einem Energietransfer durch eine Komplexbildung des verwendeten Fluorezenzfarbstoffs mit der anionischen Form eines bestimmten Indonaphthols beruht, das seinerseits einen ternären Komplex mit dem mit Kalium beladenen Ionophor bildet. Die Bestimmung von Kalium erfolgt über die Messung der veränderten Absorption nach der Beladung mit Kalium, oder auch durch Veränderung der Fluoreszenz. Die Empfindlichkeit und die Antwortzeiten dieses Systems werden als ungenügend angesehen.

Y. Kawabata beschreiben in Anal. Chem. Vol. 62, Seiten 1528- 1531 sowie 2054 bis 2055 ein Membransystem zur optischen Bestimmung von Kalium, das auf der Verwendung eines hydrophoben Ionenaustauschers, nämlich 3,6-Bis(dimethylamino)-10-dodecyl- oder -10-hexadecyl-acridiniumbromid beruht. Eine Veränderung der Fluoreszenz wird durch eine Änderung der Polarität in der Mikroumgebung der Probe erzielt, weil mittels Ionenaustausch mit dem Kaliumion die Acridiniumsalze an die Grenzfläche zur wässrigen Phase diffundieren.

W. E. Morf et al. beschreiben in Pure & Appl. Chem., Vol. 61, No. 9. Seiten 1613 bis 1618 (1989) die Verwendung von pH-sensitiven Chromo- oder Fluoroionophoren zur optischen Bestimmung von Kationen auf der Basis von Ionenaustauschreaktionen. Die Empfindlichkeit dieser Systeme ist relativ gering, die Messung in optisch dichten Systemen ist erschwert und es es werden relativ hohe Konzentrationen an Chromo- oder Fluoroionophoren in der Membran benötigt.

K. Wang et al. beschreiben in Analytical Science, Vol. 6, Seiten 715 bis 720 (1990) Membranen mit einem Absorptionsfarbstoff (Nilblau) als Indikatorsubstanz zur optischen Messung von Metallkationen. Das System beruht auf einem Ionenaustauschmechanismus, mit dem die Absorption durch die Protonierung des Farbstoffs erniedrigt wird. Die Empfindlichkeit des Systems wird als zu niedrig angesehen.

Bis heute sind keine Systeme mit einem Ionenaustauschmechanismus zur optischen Messung von Metallkationen bekannt geworden, die auf der Bestimmung der Fluoreszenzerniedrigung eines Fluorophoren beruhen und eine hohe Empfindlichkeit aufweisen, weil die Fluoreszenzquantenausbeuten und Basizitäten der bekannten pH-sensitiven Fluorophoren zu niedrig sind.

Es wurde nun gefunden, dass bestimmte Acridinfarbstoffe und Rhodaminfarbstoffe überraschend diesen hohen Anforderungen genügen und sowohl lipophile, pH-sensitive als auch stark basische Fluorophore sind, die hervorragend geeignet sind, in einer neutralen Polymermembran zusammen mit einem Ionophor und einem lipophilen Salz eines Borats zur Bestimmung von Metallkationen, besonders Kalium, gemäss dem Ionenaustauschmechanismus eine stark von den entsprechenden Metallkationenkonzentrationen abhängige Fluoreszenz aufweisen. Diese Fluorophore zeichnen sich durch eine hohe Fluoreszenzquantenausbeute, eine hohe Basizität, einen grossen Unterschied der Fluoreszenzsignale zwischen der protonierten und deprotonierten Form, eine hohe Lipophilie, eine ausreichende Photostabilität und geeignete Absorptions- und Emissionswellenlängen aus. Es können hochempfindliche Systeme zur optischen Bestimmung von Metallkationen auf der Basis von Fluoreszenzmessungen bereitgestellt werden.

Ein Gegenstand der Erfindung sind Verbindungen der Formel I und II 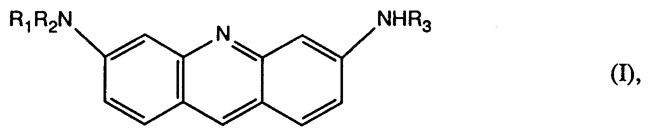 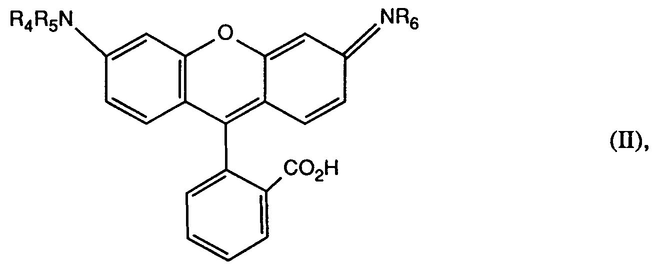
worin R₁ und R₃ sowie R₄ und R₆ für C₁-C₃₀-Alkyl oder C₁-C₃₀-Alkyl-CO- und R₂ sowie R₅ für H oder C₁-C₃₀-Alkyl stehen, mit der Massgabe, dass die Gesamtzahl der C-Atome der Alkylgruppen mindestens 12 beträgt, und deren Salze mit anorganischen oder organischen Säuren.

In einer bevorzugten Ausführungsform bedeutet R₂ H.

Die Alkylgruppen können linear oder verzweigt sein und bevorzugt 1 bis 22 C-Atome enthalten. Lineare Alkylgruppen sind bevorzugt. Beispiele für Alkyl sind Methyl, Ethyl und die Isomeren von Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Heneicosan, Docosan, Tricosan, Tetracosan, Tetracosan und Tricontan. In einer bevorzugten Ausführungsform bedeuten R₁ und R₃ C₆-C₂₄-Alkyl oder C₆-C₂₄-Alkyl-CO-, besonders bevorzugt C₁₀-C₂₄-Alkyl oder C₁₀-C₂₄-Alkyl-CO- und-insbesondere bevorzugt C₁₄-C₂₂-Alkyl oder C₁₄-C₂₂-Alkyl-CO-, wobei R₂ für H steht. In einer anderen Ausführungsform bedeuten bevorzugt R₅ H und R₄ und R₆ C₆-C₂₄-Alkyl, besonders bevorzugt C₁₀-C₂₄-Alkyl und insbesondere bevorzugt C₁₄-C₂₂-Alkyl. In einer weiteren Ausführungsform bedeuten bevorzugt R₄ und R₅ C₁-C₆-Alkyl, besonders bevorzugt C₁-C₄-Alkyl und insbesondere bevorzugt Methyl oder Ethyl, und R₆ C₁₀-C₂₄-Alkyl oder C₁₀-C₂₄-Alkyl-CO-, bevorzugt C₁₄-C₂₂-Alkyl oder C₁₄-C₂₂-Alkyl-CO- und insbesondere bevorzugt C₁₆-C₂₂-Alkyl oder C₁₆-C₂₂-Alkyl-CO-.

Die Salze der Verbindungen der Formeln I und II können sich zum Beispiel von HF, HCl, HBr, HI, H₂SO₃, H₂SO₄, H₃PO₃, H₃PO₄, HNO₂, HNO₃, HClO₄, HBF₄, HPF₆, HSbF₆, CF₃SO₃H, Toluolsulfonsäure, C₁-C₄-Alkyl- oder Phenylphosphonsäure, Ameisensäure, Essigsäure, Propionsäure, Benzoesäure, Mono- oder Di- oder Trichloressigsäure, Mono- oder Di- oder Trifluoressigsäure ableiten. Bevorzugt sind HCl, HBr, H₂SO₄, HClO₄, HBF₄, HPF₆ und HSbF₆.

Die Verbindungen der Formel I können in an sich bekannter Weise durch stufenweise Alkylierung mit verschiedenen Alkylierungsmitteln oder eine Alkylierung mit einem Alkylierungsmittel oder Acylierungsmittel des käuflichen 3,6-Diaminoacridins hergestellt werden. Geeignete Alkylierungsmittel sind zum Beispiel Dialkylsulfate oder Monohalogenalkane, besonders Chlor-, Brom- und Iodalkane. Geeignete Acylierungsmittel sind zum Beispiel Carbonsäureanhydride und besonders Carbonsäurehalogenide wie zum Beispiel Carbonsäurechloride. Diese Umsetzung kann in Gegenwart von inerten polaren und aprotischen Lösungsmitteln, zum Beispiel Ethern, alkylierten Säureamiden und Lactamen, Sulfonen oder Sulfoxiden, und bei erhöhten Temperaturen, zum Beispiel 50 bis 150 °C durchgeführt werden. Zweckmässig gibt man einen Halogenfänger zu, zum Beispiel Alkalimetallcarbonate.

Die Verbindungen der Formel II sind zum Beispiel durch die Umsetzung von Phthalsäureanhydrid mit 2 Moläquivalenten 2-Monoalkylaminophenol erhältlich. Eine andere Herstellungsmöglichkeit besteht in der Umsetzung von 2-Monoalkylaminophenol mit einem Moläquivalent 2-Hydroxy-4-dialkylamino-2'-carboxyl-benzophenon. Diese Umsetzungen sind zum Beispiel in der US-A-4 622 400 beschrieben. Zweckmässig führt man die Reaktion in einem inerten Lösungsmittel, zum Beispiel Kohlenwasserstoffen oder Ethern durch. Vorteilhaft werden molare Mengen eines Kondensationsmittels zugegeben, zum Beispiel Lewissäuren, konzentrierte Schwefelsäure, Perchlorsäure oder Phosphorsäure. Die Reaktionstemperaturen können zum Beispiel 50 bis 250 °C betragen.

Die Verbindungen der Formel I können in üblicher Weise durch Fällung, Kristallisation oder Extraktion isoliert und gegebenenfalls mittels Umkristallisation oder chromatographischer Methoden gereinigt werden. Es handelt sich um kristalline rote, rotbraune oder rotviolette Verbindungen.

Die Verbindungen der Formeln I und II eignen sich hervorragend als fluorophore Farbstoffindikatoren für die optische Bestimmung von Kationen in wässriger Umgebung insbesondere durch Messung der Veränderung der Fluoreszenz.

Ein weiterer Gegenstand der Erfindung ist eine Zusammensetzung aus
(a) einem transparenten Träger,
(b) der auf mindestens einer Seite mit einer transparenten Schicht beschichtet ist, die
   (b1) ein hydrophobes Polymer
   (b2) einen Weichmacher,
   (b3) das Salz eines lipophilen Anions
   (b4) einen Ionophor, der mit dem zu bestimmenden Ion einen Komplex bildet, und
   (b5) eine Verbindung der Formel I oder II als Fluorophor enthält.

Die Verbindungen der Formeln I und II weisen bevorzugt einen pKₐ-Wert von mindestens 8, besonders bevorzugt mindestens 10 auf.

Der Träger kann zum Beispiel aus einem Kunststoffmaterial, mineralischen Materialien oder Glas gebildet sein und von beliebiger Gestalt sein, zum Beispiel Platten, Zylinder, Rohre, Bänder oder Fasern. Bevorzugt sind Gläser.

Die Dicke der Schicht auf dem Träger kann zum Beispiel von 0,01 bis 100 µm, bevorzugt 0,1 bis 50 µm, bevorzugter 0,1 bis 30 µm und besonders bevorzugt 0,1 bis 10 µm betragen.

Für die Zusammensetzung sind verschiedenartige Polymere geeignet. Zweckmässig weisen sie ein mittleres Molekulargewicht von mindestens 100 000 Dalton auf, zum Beispiel 100 000 bis 2 000 000 Dalton, bevorzugt 200 000 bis 1 000 000 Dalton. Die Polymere müssen eine ausreichende Löslichkeit in organischen Lösungsmitteln besitzen, damit sie mit den anderen Komponenten vermischt und mit üblichen Beschichtungsverfahren zu Schichten verarbeitet werden können. Einige Beispiele für Homo- und Copolymere sind solche aus Olefinen, Acrylaten, Methacrylaten, Vinylestern, Acrylnitril, Dienen, Styrol, Methylstyrol, Vinylchlorid, Vinylfluorid, Vinylidenchlorid, Vinylethern. Einige spezifische Beispiele sind Polyvinylchlorid, Polyvinylidenchlorid, Polyvinylfluorid, Polyacrylnitril, Polystyrol, Poly(methylstyrol), Polyacrylate und Polymethacrylate mit C₁-C₁₈-Alkylresten in den Estergruppen, Vinylchlorid/Vinylacetat-Copolymere, Vinylchlorid/Vinylace- tat/Vinylalkohol-Copolymere, Vinylchlorid/Vinylacetat/Acrylnitril-Copolymere, Vinylchlorid/Vinylidenchlorid-Copolymere, Vinylidenchlorid/Acrylnitril-Copolymere, Acrylnitril/Butadien/Styrol-Copolymere. Bevorzugte Polymere sind Homopolymere aus Vinylchlorid und Vinylidenchlorid sowie Copolymere Vinylchlorid und/oder Vinylidenchlorid und Acrylaten, Methacrylaten, Vinylestern, Vinylalkohol, Acrylnitril und Styrol. Besonders bevorzugt ist Polyvinylchlorid.

Es ist vorteilhaft, den Polymeren einen Weichmacher einzuverleiben, um die Diffusionskoeffizienten der Membrankomponenten für den Ionenaustausch zu optimieren. Je nach Art der Polymere und Weichmacher können 10 bis 90 Gew.-%, bevorzugt 20 bis 70 und besonders bevorzugt 20 bis 50 Gew.-% Polymer und 90 bis 10, bevorzugt 80 bis 30 und insbesondere 80 bis 50 Gew.-% Weichmacher enthalten sein. Geeignete Weichmacher sind in grosser Vielzahl bekannt. Es kann sich zum Beispiel um höhere Alkanole oder deren Ester, Ester von Fettsäuren mit Diolen oder Alkanolen, Ether mit höheren Alkanolen, Ester von Di- und Polycarbonsäuren und Ester von höheren Alkanolen und Phosphorsäure oder phosphoriger Säure handeln. Die höheren Alkanole können zum Beispiel lineare oder verzweigte C₆-C₂₂-Alkanole oder mit 1 bis 3 linearen oder verzweigten C₃-C₁₈-Alkyl- oder C₃-C₁₈-Alkoxygruppen substituierte Phenole sein. Einige spezifische Beispiele sind Octadecanol, Phthalsäurediester, Glutarsäurediester, Adipinsäurediester, Azelainsäurediester und Sebacinsäurediester mit C₈-C₁₈-Alkanolen wie 2-Ethylhexanol, n-Octanol, n-Decanol, n-Dodecanol, Tetradecanolen, Hexadecanolen Heptadecanolen und Octadecanolen, Tris(2-ethylhexyl)phosphat oder Tris(2-ethylhexyl)phosphit, Tris(nonylphenyl)phosphit, Ethylenglykolmonostearat, Ethylenglykoldibenzoat und 2-Nitrophenylbutyl- oder -octylether. Die Weichmacher werden zweckmässig so ausgewählt und in solchen Mengen eingesetzt, dass sie mit dem Polymer verträglich sind, keine Phasentrennungen auftreten und die hydrophoben Eigenschaften des Polymers nicht oder nur wenig verändern. Die Weichmacher sollen lipophil sein und tragen dazu bei, die Komponenten in der Schicht (Matrix) zu lösen und zu verteilen.

Als Salze mit lipophilen Anionen sind zum Beispiel Alkalimetall-, Erdalkalimetall- und Ammoniumsalze mit gegebenenfalls substituierten Tetraphenylboraten geeignet. Bevorzugte Kationen sind Li^{⊕}, Na^{⊕}, K^{⊕}, Mg^{2⊕}, Ca^{2⊕}, NH₄^{⊕}, und die Ammoniumkationen von primären, sekundären und tertiären Aminen sowie quaternäre Ammoniumkationen, die 1 bis 60 C-Atome enthalten können. Einige Beispiele für Ammoniumkationen sind Methyl-, Ethyl-, Propyl-, Butyl-, Hexyl-, Octyl-, Decyl-, Dodecyl-, Tetradecyl-, Hexadecyl-, Octadecyl-, Dimethyl-, Diethyl-, Dibutyl-, Butyl-methyl-, Dioctyl-, Didoceyl-, Dodecyl-methyl-, Trimethyl-, Triethyl-, Tripropyl-, Tributyl-, Trioctyl-, Tridodecyl-, Dodecyldimethyl-, Didoecyl-methyl-, Tetramethyl-, Tetraethyl-, Tetrapropyl-, Tetrabutyl-, Tetrahexyl-, Tetraoctyl-, Tetradecyl-, Tetradodecyl-, Dodecyl-trimethyl-, Octyl-trimetyl-, Didodecyl-dimethyl-, Tridodecyl-methyl-, Tetradecyl-trimethyl- und Octadecyl-trimethylammonium. Quaternäre Ammoniumsalze sind bevorzugt, besonders solche mit 4 bis 48 C-Atomen.

Als Boratanion ist zum Beispiel Tetraphenylborat geeignet, dessen Phenylgruppen mit ein oder mehreren, bevorzugt 1 bis 3 und besonders bevorzugt 1 oder 2 C₁-C₄-Alkyl, C₁-C₄-Alkoxy, Halogen wie zum Beispiel F oder Cl, oder Trifluormethyl substituiert sein können. Einige spezifische Beispiele sind Natriumtetraphenylborat, Natriumtetra(3,5-bis-trifluormethylphenyl)borat, Kaliumtetra(4-chlorphenyl)borat, Tetrabutylammonium-tetraphenylborat und Tetradodecyl(4-chlorphenyl)borat. Die Salze mit lipophilen Anionen dienen als negativer Ladungsausgleich für in die aktive Schicht diffundierende und dort komplexierte zu messende Metallkationen.

Die Menge an Salzen mit lipophilen Anionen kann zum Beispiel 0,01 bis 10 Gew.-%, bevorzugt 0,1 bis 5 Gew.-% und besonders bevorzugt 0,1 bis 2 Gew.-% betragen, bezogen auf die Menge an Polymer und Weichmacher.

Die Polymerschicht (auch als Membran bezeichnet) enthält einen Ionophor zum Beispiel in einer Menge von 0,01 bis 10 Gew.-%, bevorzugt 0,1 bis 5 Gew.-% und besonders bevorzugt 0,1 bis 2 Gew.-%, bezogen auf die Menge an Polymer und Weichmacher. Ionophore sind organische natürliche oder synthetische Verbindungen, die mehrere meist alternierende elektronenreiche Heteroatome wie zum Beispiel S, N und besonders O in einer offenkettigen oder cyclischen Kohlenstoffkette enthalten und die zu messenden Metallkationen selektiv zu komplexieren vermögen. Bei den natürlichen Verbindung handelt es sich häufig um makrozyklische Verbindungen wie zum Beispiel Valinomycin, das selektiv Kaliumkationen zu binden vermag. Ein anderes Beispiel ist Nonactin. Eine grosse Gruppe von Ionophoren sind die makrocylischen Polyether (Kronenether), die je nach Geometrie und Grösse unterschiedliche Metallkationen zu komplexieren vermögen. Als andere Beispiele für Ionophore sind Coronandene, Kryptandene und Calixarene zu nennen. Ein Beispiel für offenkettige sind die Podandene. Solche Ionophore sind zum Beispiel in der US-A-4 645 744 beschrieben.

Die Menge an Verbindungen der Formeln I und II kann zum Beispiel 0,01 bis 10 Gew.-%, bevorzugt 0,1 bis 5 Gew.-% und besonders bevorzugt 0,1 bis 2 Gew.-% betragen, bezogen auf die Menge an Polymer und Weichmacher. Die Verbindungen der Formeln I und II können auch über geeignete Brückengruppen an die Polymeren gebunden sein.

Die erfindungsgemäss zu verwendenden Fluorophore weisen sehr geeignete Absorptions- und Emmissionswellenlängenbereiche auf, die die Anwendung bekannter und preiswerter Lichtquellen und Detektoren erlauben, zum Beispiel Halogen- oder Xenonlampen oder Licht emittierende Dioden. Als Detektoren können zum Beispiel Photodioden eingesetzt werden. Die Fluorophore besitzen ferner hohe Extinktionskoeffizienten und es können hohe Quantenausbeuten erzielt werden. Die hohe Lipohilie, hohe Basizität und der grosse dynamische Bereich der Fluoreszenzänderung zwischen protonierter und deprotonierter Form erfüllen besonders die hohen Anforderungen einer optischen Bestimmung von Kationen auf der Basis von Fluoreszenzmessungen.

Als Kationen kommen zum Beispiel Metallkationen der Metalle aus den ersten bis fünften Hauptgruppen, den ersten bis achten Nebengruppen des Periodensystems der Elemente, den Lanthaniden und Actiniden in Frage. Einige Beispiele für Metalle sind Li, Na, K, Rb, Cs, Mg, Ca, Sr, Ba, B, Al, Ga, In, Tl, Sn, Pb, Sb, Bi, Cu, Ag, Au, Zn, Cd, Hg, Sc, Y, Ti, Zr, Hf, Cr, Mo, W, Mn, Fe, Co, Ni, Ru, Os, Rh, Ir, Pt, Pd, La, Ce, Pr, Nd, Pm, Sm, Eu, Gd, Tb, Dy, Ho, Er, Yb, Lu, Ac, Th, Pa, U, Np, Pu. Bevorzugte Metallkationen sind die Alkali- und Erdalkalimetallionen, besonders Li^{⊕}, Na^{⊕}, K^{⊕}, Mg^{2⊕}, Ca^{2⊕} und Sr^{2⊕}, und ganz besonders K^{⊕}, Na^{⊕} und Ca^{⊕}. Als Ammoniumkationen sind zum Beispiel NH₄^{⊕} sowie die Kationen protonierter primärer, sekundärer und tertiärer Amine als auch quaternäres Ammonium geeignet. Die Amine können 1 bis 40, bevorzugt 1 bis 20 und besonders bevorzugt 1 bis 12 C-Atome enthalten. Das quaternäre Ammonium kann 4 bis 40, bevorzugt 4 bis 20 und besonders bevorzugt 4 bis 16 C-Atome enthalten.

Die erfindungsgemässe Zusammensetzung eignet sich hervorragend als optischer Sensor zur quantativen Bestimmung von Kationen, besonders Metallkationen, ganz besonders Kaliumkationen, in wässriger Umgebung mittels bevorzugt Fluoreszenzspektrometrie. Die Bestimmungen können in kurzen Zeiten bei hoher Genauigkeit auch bei kleinen Konzentrationen (zum Beispiel im µ-molaren Bereich bis zum nanomolaren Bereich) vorgenommen werden, da sich die pH-abhängigen Gleichgewichte der Komplexierungsreaktionen und des Protonenaustausches schnell einstellen und die Fluorophore durch eine hohe Fluoreszenzquantenausbeute und Empfindlichkeit gekennzeichnet sind. Die Analysen können zum Beispiel direkt in Körperflüssigkeiten (Blut, Urin, Serum), Naturgewässern oder Abwässern vorgenommen werden, wobei möglicherweise störende Kationen zuvor spezifisch gebunden oder entfernt werden können. Die erfindungsgemässe Zusammensetzung eignet sich besonders zur Bestimmung physiologischer Mengen von Kationen in wässrigen Medien, die etwa im Bereich von 0,5 bis 10 mMol liegen können.

Neben der bevorzugten Fluoreszenzspektroskopie können auch andere optische Messmethoden angewendet werden, zum Beispiel Oberflächenplasmonresonazspektroskopie, Absorptionsspektroskopie, Reflexionsspektroskopie, Interferometrie oder oberflächenverstärkte Raman- oder Fluoreszenzspektroskopie.

Ein weiterer Gegenstand der Erfindung ist ein optischer Sensor für die Bestimmung von Kationen in wässrigen Messproben besonders mittels Fluoroszenzspektrometrie, der aus
(a) einem transparenten Träger besteht,
(b) der auf mindestens einer Seite mit einer transparenten Schicht beschichtet ist, die
   (b1) ein hydrophobes Polymer
   (b2) einen Weichmacher,
   (b3) das Salz eines lipophilen Anions
   (b4) einen Ionophor, der mit dem zu bestimmenden Ion einen Komplex bildet, und
   (b5) eine Verbindung der Formel I oder II als Fluorophor enthält.

Ein anderer Gegenstand der Erfindung ist ein Verfahren zur optischen Bestimmung von Kationen in wässrigen Messproben, bei dem man eine erfindungsgemässe Zusammensetzung mit besagter wässriger Messprobe in Kontakt bringt und dann insbesondere die Erniedrigung der Fluoreszenz in der aktiven Polymerschicht misst.

Ein weiterer Gegenstand der Erfindung ist die Verwendung der Verbindungen der Formeln I und II als Fluorophore zur optischen Bestimmung von Kationen in wässrigen Messproben.

Das erfindungsgemässe Verfahren kann zum Beispiel so durchgeführt werden, dass man die erfindungsgemässe Zusammensetzung aus Träger und aktiver Polymerschicht in einer optischen Zelle befestigt, in der die aktive Schicht mit der Messprobe in Kontakt kommt. Die optische Zelle enthält ein Fenster, durch das die aktive Schicht zur Anregung bestrahlt und die emittierte Fluoreszenzstrahlung mit einem Spektrofluorometer gemessen werden kann. Die Wellenlängen werden so eingestellt, dass für die Bestrahlung das Absorptionsmaximum und für die Fluoreszensmessung das Emissionsmaximum vorliegt. Gemessen wird die Intensitätsabnahme in Abhänigkeit von der Zeit. Das Messystem kann so ausgebildet sein, dass die Messung diskontinuierlich oder kontinuierlich erfolgt, indem man zum Beispiel die Messlösung durch die Messzelle pumpt. Zur Bestimmung unbekannter Konzentrationen an Kationen wird das System mit Messproben bekannter Konzentration zunächst geeicht, indem man die Konzentrationen in Abhängigkeit von der Intensität der Fluoreszenz aufträgt. Der Messprobe werden zweckmässig pH-Puffer zugegeben, da die Empfindlichkeit der Messung wegen der pH-Abhängigkeit des Absorptionsspektrums und folglich auch die Fluoreszenzintensität des Fluorophors vom pH der Messlösung abhängt. Der pH-Bereich der Messprobe kann zum Beispel 4 bis 8, bevorzugterweise 5,5 bis 6,5 betragen. Geeignete Puffer sind zum Beispiel Citratpuffer und Phosphatpuffer. Weitere Puffersysteme sind in der US-A-4 645 744 beschrieben, insbesondere auch solche, die direkt der aktiven Schicht einverleibt werden, um die Zugabe zur Messprobe zu vermeiden.

Die nachfolgenden Beispiele erläutern die Erfindung näher.

### A) Herstellung von Fluorophoren

### Beispiel A1: Herstellung von 3,6-Bis(n-octylamino)-acridin.

Eine Lösung von 2,5 g 3,6-Diaminoacridin-hdrochlorid und 3,55 ml 1-Bromoctan in 50 ml Dimethylsulfoxid wird mit 6,33 g wasserfreiem Kaliumcarbonat versetzt und 48 h bei 80 °C gerührt. Anschliessend wird das abgekühlte Reaktionsgemisch auf Eis gegossen und die braune Suspension mit Methylenchlorid extrahiert. Die organische Phase wird mit gesättigter wässriger NaCl-Lösung gewaschen und über Natriumsulfat getrocknet. Nach dem Einengen wird das rotbraune Öl an Kieselgel mit Methylenchlorid/Methanol (9:1) chromatographiert. Nach dem Abdampfen des Lösungsmittels wird der Rückstand mit Diethylether/Methanol (10:1) aufgenommen und an Aluminiumoxid chromatographiert. Das Eluat wird in Methanol aufgenommen und mit 2N HCl versetzt, mit Diethylether extrahiert, die Etherphase getrocknet und eingedampft. Der Rückstand wird in Methylenchlorid gelöst, mit n-Hexan versetzt und der gebildete rote kristalline Niederschlag abfiltiert. Aus der Mutterlauge kann nach dem Einengen weiteres Produkt isoliert werden. Der Schmelzpunkt der Titelverbindung beträgt 245 °C. Absorptionsspektrum (Ethanol): λₘₐₓ= 472 nm; ε = 51 400.

### Beispiel A2: Herstellung von 3,6-Bis(n-eicosylamino)-acridin.

Eine Lösung von 2,5 g 3,6-Diaminoacridin-hdrochlorid und 2,95 g 1-Eicosylbromid in 20 ml N,N'-Dimethyl-ethylenharnstoff wird mit 2,53 g wasserfreiem Kaliumcarbonat versetzt und 86 h bei 50 °C gerührt. Anschliessend wird das abgekühlte Reaktionsgemisch auf Wasser gegossen und die orange-braune Suspension mit Methylenchlorid extrahiert. Die organische Phase wird mit Wasser gewaschen und über Natriumsulfat getrocknet. Nach dem Einengen wird das braune Öl mit 2N HCl versetzt. Der gebildete rote Niederschlag wird abfiltriert, mit Wasser gewaschen und dann im Hochvakuum getrocknet. Die erhaltenen rotbraunen Kristalle werden in Methylenchlorid/Methanol (10:1) aufgenommen und an Kieselgel chromatographiert. Nach dem Einengen wird mit Diethylether/Methanol (10:1) aufgenommen und erneut an Kieselgel chromatographiert. Man erhält die Titelverbindung als rote Kristalle, Absorptionsspektrum (Ethanol): λₘₐₓ= 472 nm; ε = 42 200.

### Beispiel A3: Herstellung von 3,6-Bis(n-hexylamino)-acridin.

Eine Lösung von 500 mg N,N'-Bis-tosyl-3,6-diaminoacridin und 797 mg 1-Bromhexan in 25 ml Dimethylformamid wird mit 298 mg gemahlenem Kaliumhydroxid versetzt und 22 h bei 60 °C gerührt. Anschliessend wird das abgekühlte Reaktionsgemisch auf Wasser gegossen, mit Essigsäureethylester extrahiert, die abgetrennte organische Phase mit wässriger NaCl-Lösung gewaschen und dann über Natriumsulfat getrocknet. Nach dem Einengen erhält man ein dunkelrotes Öl, das in Toluol/Essigsäureethylester (20:1) aufgenommen und an Kieselgel chromatographiert wird. Nach dem Abdampfen des Lösungsmittels erhält man ein gelbes viskoses Öl, das man in 11,5 ml Eisessig löst und bei Wasserkühlung mit 4,6 ml 97-%iger Schwefelsäure versetzt und danach 15 h bei Raumtemperatur rührt. Das rote Reaktionsgemisch wird in Eiswasser gegossen und mit 30-%iger NaOH auf pH 11 gestellt. Man extrahiert mit Essigsäureethylester, wäscht die organische Phase mit 2N HCl und gesättigter wässriger NaCl-Lösung und trocknet danach über Natriumsulfat. Nach dem Einengen wird das dunkelrote viskose Öl mit t-Butyl-methylether/Methanol (5:1) aufgenommen und an Kieselgel chromatographiert. Man erhält die Titelverbindung als orangerote Kristalle mit einem Schmelzpunkt von > 200 °C (Zersetzung). ¹H-NMR (CDCl₃): 8,1 [s, 1H, C(9)]; 7,44 [d, 2H, C(8)]; 6,93 [s, 2H, C(5)]; 6,82 [d, 2H, C(7)]; 3,20 [t, 4H, N-CH₂]; 1,68 [m, 6H, CH₃].

### Beispiel A4: Herstellung von 3,6-Bis(n-heptylcarbonylamino)-acridin.

Zu einer Suspension von 2,5 g 3,6-Diaminoacridin-hydrochlorid in 50 ml Pyridin werden langsam 3,1 ml Heptanoylchlorid zugetropft und danach 30 min gerührt. Anschliessend wird das Reaktionsgemisch auf Wasser gegossen. Die gelbe Suspension wird mit Methylenchlorid extrahiert, die organische Phase mit wässriger gesättigter NaCl-Lösung gewaschen und über Natriumsulfat getrocknet. Nach dem Einengen wird das dunkelrote Öl in Methylenchlorid/Methanol (10:1) aufgenommen und an Kieselgel chromatographiert. Das eingengte Eluat wird in Methylenchlorid aufgenommen und Cyclohexan zugetropft. Der gebildete gelbe Niederschlag wird abfiltriert, mit Cyclohexan gewaschen und im Hochvakuum getrocknet. Man erhält die Titelverbindung als gelbe Kristalle mit einem Schmelzpunkt von 243-244 °C. Absorptionsspektrum (Ethanol): λₘₐₓ= 384 nm; ε = 2 300.

### Beispiel A5: Herstellung von

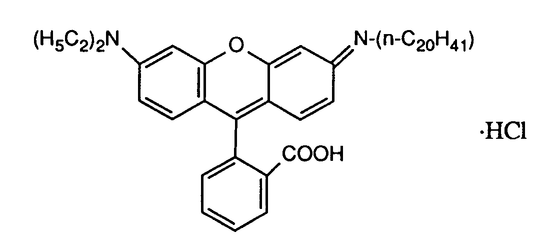

a) Eine Lösung aus 12,8 g Phthalsäureanhydrid und 13,2 g 3-N,N-Diethylaminophenol wird in 75 ml Toluol 16 h bei 110 °C gerührt. Das ausgefallene Produkt wird abfiltriert und in Etanol umkristallisiert. Man erhält ziegelrote Kristalle von 1-Carboxyl-1'-Hydroxyl-3'-diethylamino-benzophenon (Produkt A) mit einem Schmelzpunkt von 214 °C.
b) Eine Lösung aus 5,5 g 3-Aminophenol und 21,6 g 1-Bromeicosan wird in 250 ml 1,4-Dioxan 48 h bei 100 °C gerührt. Man dampft im Vakuum ein, nimmt den braunen gelartigen Rückstand in Toluol/Essigsäureethylester (10:1) auf und chromatographiert an Kieselgel. Man erhält 3-N-Eicosylaminophenol als weisse Kristalle mit einem Schmelzpunkt von 80 °C.
c) 626 mg Produkt A und 790 mg 3-N-Eicosylaminophenol werden in 5 ml Phosphorsäure (85 %-ig) bei 170 °C während 2 h gerührt.Nach dem Abkühlen versetzt man mit einer Lösung von 1 ml konzentrierter HCl in 1 ml Methanol und extrahiert anschliessend mit Methylenchlorid. Nach Entfernen des Lösungsmittels wird der Rückstand in Methylenchlorid/Methanol (85:15) aufgenommen und an Kieselgel chromatographiert. Man erhält die Titelverbindung als rot-violette Kristalle mit einem Schmelzpunkt von 115 °C. Absorptionsspektrum (Ethanol): λₘₐₓ= 532 nm; ε = 90 000.

### Beispiel A6: Herstellung von

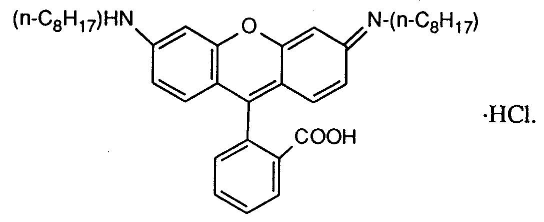

a) Eine Lösung von 5,45 g 3-Aminophenol und 11,6 g 1-Bromoctan in 250 ml Dioxan wird 80 h bei 100 °C gerührt, dann das Lösungsmittel abgedampft, der Rückstand dann in Toluol/Essigsäureethylester (10:1) aufgenommen und an Kieselgel chromatographiert. Man erhält N-Octylaminophenol als beige Kristalle, Schmelzpunkt 75 °C.
b) 1,1 g N-Octylaminophenol und 0,37 g Phthalsäureanhydrid werden bei 100 °C zusammen geschmolzen. Zur Schmelze gibt man 1 ml Phosphorsäure (85 %-ig) und erwärmt auf 170 °C. Nach 1 h lässt man abkühlen und versetzt mit 2N HCl. Man extrahiert mit Methylenchlorid, entfernt das Lösungsmittel und nimmt den roten Rückstand mit Methylenchlorid/Methanol (85:15) auf. Die Chromatographie an Kieselgel ergibt die Titelverbindung als rote Kristalle mit einem Schmelzpunkt von 183 °C. Absorptionsspektrum (Ethanol): λₘₐₓ= 522 nm; ε = 73 700.

### Beispiel A7: Herstellung von

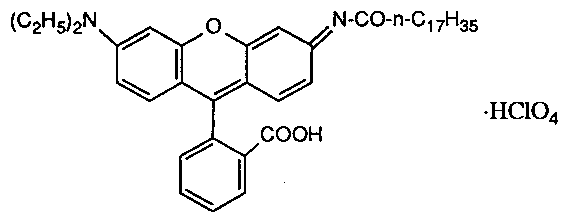

a) 1,57 g Produkt A aus Beispiel A5a, 0,55 g 3-Aminophenol und 10 ml Phosphorsäure (85 %-ig) werden bei 170 °C 30 min gerührt. Darauf werden 6,7 ml Perchlorsäure (50 %-ig) und 100 ml Methanol zugegeben, erneut erwärmt und danach im Vakuum das Lösungsmittel entfernt. Der Rückstand wird wird in Methylenchlorid aufgenommen, mit Wasser gewaschen und das Lösungsmittel wieder entfernt. Der Rückstand wird in Methylenchlorid/Methanol (10:1) aufgenommen und an Kieselgel chromatographiert. Man erhält rote Kristalle der Verbindung B der Formel 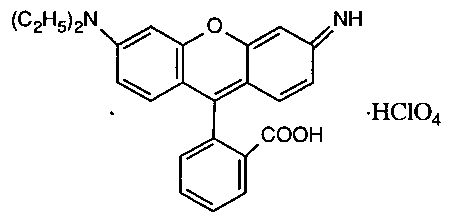 mit einem Schmelzpunkt von 175 °C.
b) 0,1 g der Verbindung B werden in ein ml Methylenchlorid und 0,3 ml Pyridin gelöst und mit 100 mg Stearoylchlorid versetzt. Nach 3 h wird im Vakuum zur Trockene eingedampft, der Rückstand in Methylenchlorid/Methanol (85:15) gelöst und an Kieselgel chromatographiert. Man erhält die Titelverbindung als rote Kristalle mit einem Schmelzpunkt von 145 °C. Absorptionsspektrum (Ethanol): λₘₐₓ= 56 nm; ε = 10 900.

### B) Herstellung von beschichteten Trägern

### Beispiel B1-B6:

a) Als Trägermaterial wird vorbehandeltes Glas verwendet. Runde Glasscheiben (Durchmesser 18 mm, Dicke 0,17 mm) werden eine Stunde in eine Lösung von 10 Vol-% Dimethyl-dodecylchlorsilan in Isopropanol getaucht. Danach wäscht man die Glasscheiben nacheinander mit je 200 ml Isopropanol, Ethanol und Methanol und trocknet 1 h bei 110 °C. Die hydrophobisierte Oberfläche weist eine verbesserte Haftung der Membranschicht auf.
b) Herstellung der Beschichtungslösung.
Die folgenden Bestandteile werden zusammen mit 1,5 ml Tetrahydrofuran in eine 2 ml Flasche gegeben und bis zur Lösung der Komponenten geschüttelt:
1. 80 mg Polyvinylchlorid (Fluka, 81392)
2. 160 mg Bis-(2-ethylhexyl)sebacat (Weichmacher)
3. 5 mg Valinomycin
4. 1,5 mg Kalium-tetrakis(4-fluorphenyl)borat
5. 2 mg Fluorophor

| Beispiel Nr. | Fluorophor |
|---|---|
| B1 | Beispiel A5 |
| B2 | Beispiel A6 |
| B3 | Beispiel A7 |
| B4 | Beispiel A1 |
| B5 | Beispiel A2 |
| B6 | Beispiel A4 |

c) Herstellung von beschichteten Glasträgern.
Die Glasträger werden in der Kammer eines Schleuderbeschichtungsapparats (Optocoat OS 35var, Willer Company, CH-8484 Weisslingen) befestigt. Die Kammer wird mit 10 ml Tetrahydrofuran gespült und 2 min bei 3800 Umdrehungen/Minute rotiert. Danach werden 50 µl der jeweiligen Beschichtungslösung auf den Glasträger pipettiert und der Glasträger noch 10 sec rotiert. Man entfernt den mit einer Membran beschichteten Glasträger und trocknet ihn 10 min an der Luft.
d) Spektrale Eigenschaften der Membranen.
Die beschichteten Glasträger werden in einer optischen Zelle befestigt, in der die Membran mit der Messflüssigkeit in Berührung steht. Die Membran kann in der optischen Zelle optisch angeregt und die Fluoreszenzstrahlung gemessen werden. Die optische Zelle wird in ein Spektrofluorometer (Pekin-Elmer LS-50) gebracht und das Absorptions- und Emmissionsspektrum gemessen. Ferner wird die relative Fluoreszenzquantenausbeute gemäss der von Calvert und Pitts in Photochemistry, Verlag: John Wiley and Sons Inc., Seiten 799 bis 805 (1966) beschriebenen Methode mit Fluorescein als Standard bestimmt. Die Ergebnisse sind in Tabelle 1 angegeben.

**Tabelle 1:**

| Beispiel | Absorption (λₘₐₓ, nm) | Emmission (λₘₐₓ, nm) | Extinktionskoeffizient | Quantenausbeute |
|---|---|---|---|---|
| B1 | 540 | 570 | 90 000 | 0,14 |
| B2 | 525 | 555 | 73 700 | 0,33 |
| B3 | 500 | 560 | 20 000 | 0,08 |
| B4 | 470 | 505 | 51 400 | 0,75 |
| B5 | 470 | 505 | 42 200 | 0,75 |
| B6 | 380 | 460 | 2 300 | 0,10 |

### Beispiele B7 bis B12: Bestimmung von pKa-Werten und der Fluoroszenzänderung.

Die Fluorophore werden in einem Gemisch aus 30 Vol.-% Methanol und 70 Vol.-% Phosphatpuffer gelöst und verschiedene pH-Werte zwischen 6 und 13 eingestellt. Die Messlösungen werden in eine Küvette gefüllt und die Fluoreszenzintensität mit einem Spektrophotometer gemessen. Die Fluoreszenzintensität wird als Funktion des pH-Wertes aufgetragen und aus dem Wendepunkt der Kurve der pKₐ-Wert ermittelt. Ferner wird das Verhältnis von protonierter und deprotonierter Form des Fluorophors durch die Fluoreszenzänderung bestimmt, indem man den Fluorophor in Tetrahydrofuran löst, den ph-Wert durch Zugabe von 1 M HCl beziehungsweise 1,0 M NaOH einstellt und die Änderung der Fluorezensintensität in Prozent bestimmt. Die Konzentration der Fluorophore beträgt jeweils 10⁻⁷ mol/l. Die Ergebnisse sind in Tabelle 2 angegeben.

**Tabelle 2:**

| Fluorophor Beispiel | pKₐ-Wert | Änderung Fluoreszenzintensität |
|---|---|---|
| A1 | 10,0 | 44 |
| A2 | 11,1 | 41 |
| A4 | 10,0 | 24 |
| A5 | 10,3 | 33 |
| A6 | 12,0 | 20 |
| A7 | 10,1 | 15 |

### C) Anwendungsbeispiele

### Beispiel C1:

Es wird die gleiche Versuchsanordnung wie in Beispiel B1d verwendet und die Absorptions- sowie Emmissionswellenlängen auf die entsprechenden Maxima der in der Membran eingesetzten Fluorophore eingestellt. Die Membran wird mit einer wässrigen KCl-Lösung definierter Konzentration in Kontakt gebracht, indem man die Lösung mit einer Geschwindigkeit von 1 ml/min durch die Zelle pumpt und die Änderung der Fluoreszenzintensität bestimmt. Vor der Messung und nach jeder Messung wird mit Kaliumionen-freien Pufferlösungen gespült und die Fluoreszenzintensität bestimmt, um die Basislinie festzulegen. Die Erniedrigung der Fluoreszenzintensität in Prozent bei der jeweiligen Kaliumkonzentration für den Fluorophor gemäss Beispiel A5 (Membran B1) sind in der Tabelle 3 angegeben.

**Tabelle 3:**

| Kalium-Konzentration (mM) | % Erniedrigung Fluoreszenzintensität |
|---|---|
| 0,01 | 5 |
| 0,1 | 20 |
| 0,5 | 35 |
| 1,0 | 45 |
| 5,0 | 53 |
| 10,0 | 60 |
| 100,0 | 68 |

Aus der Tabelle ist ersichtlich, dass die Fluoreszenzintensität mit steigender Kaliumkonzentration abnimmt und nach einer Eichung unbekannte Konzentrationen mit hoher Zuverlässigkeit bestimmt werden können. Insbesondere im physiologischen Bereich von etwa 0,5 bis 10 mM Kalium ist eine hohe Empfindlichkeit gewährleistet.

### Beispiel C2:

Es wird wie in Beispiel C1 verfahren unter Verwendung des Fluorophors gemäss Beispiel A6. Die Erniedrigung der Fluoreszenzintensität in Prozent bei der jeweiligen Kaliumkonzentration für den Fluorophor gemäss Beispiel A6 (Membran B2) sind in der Tabelle 4 angegeben.

**Tabelle 4:**

| Kalium-Konzentration (mM) | % Erniedrigung Fluoreszenzintensität |
|---|---|
| 0,1 | 36 |
| 0,5 | 41 |
| 1,0 | 43 |
| 5,0 | 48 |
| 10,0 | 52 |

### Beispiel C3:

Es wird wie in Beispiel C1 verfahren unter Verwendung des Fluorophors gemäss Beispiel A1. Die Erniedrigung der Fluoreszenzintensität in Prozent bei der jeweiligen Kaliumkonzentration für den Fluorophor gemäss Beispiel A1 (Membran B4) sind in der Tabelle 5 angegeben.

**Tabelle 5:**

| Kalium-Konzentration (mM) | % Erniedrigung Fluoreszenzintensität |
|---|---|
| 0,1 | 5 |
| 0,5 | 15 |
| 1,0 | 25 |
| 5,0 | 39 |
| 10,0 | 45 |
| 100,0 | 75 |

### Beispiel C4:

Es wird wie in Beispiel C1 verfahren und die Natriumionenkonzentration mit einer Membran aus 2 mg Fluorophor gemäss Beispiel A5, 30 mg Natriumionophor (4-Octadecanoyloxymethyl-N,N,N,N-tetracyxlohexyl- 1 ,2-phenylendioxy-diacetamid, Fluka Natrium Ionophor V, Katalog Nr. 71738), 2 mg Kalium-tetrakis(4-chlorphenyl)borat, 75 mg Polyurethan (Tecoflex®), 160 mg Bis-2-ethylhexyl-sebacat als Weichmacher und 1 ml Tetrahydrofuran bestimmt. Der Sensor wird einer Pufferlösung bei pH 5 (0,1 Mol Tris-hydroxymethyl-aminomit-methan und 1M HCl) unterschiedlichen Natriumkonzentrationen ausgesetzt. Die Ergebnisse sind in Tabelle 6 angegeben.

**Tabelle 6:**

| Natrium-Konzentration (mM) | % Erniedrigung Fluoreszenzintensität |
|---|---|
| 0 | 0 |
| 3 | 15 |
| 15 | 41 |
| 30 | 54 |
| 90 | 72 |
| 150 | 79 |
| 210 | 83 |
| 300 | 86 |

### Beispiel C5:

Es wird wie in Beispiel C1 verfahren und die Kalziumionenkonzentration mit einer Membran aus 2 mg Fluorophor gemäss Beispiel A5, 30 mg Kalziumionophor ((-)-(R,R)-N,N-[Bis(11-ethoxycarbonyl)undecyl]-N,N-4,5-tetramethyl-3,6-dioxaoctandia mid, Fluka Kalzium Ionophor I, Katalog Nr. 21192), 6 mg Kalium-tetrakis(4-chlorphenyl)borat, 75 mg Polyurethan (Tecoflex®), 160 mg Bis-2-ethylhexyl-sebacat als Weichmacher und 1 ml Tetrahydrofuran bestimmt. Der Sensor wird einer Pufferlösung bei pH 5 (0,02M NaOH und Essigsäure) unterschiedlichen Kalziumkonzentrationen ausgesetzt. Die Ergebnisse sind in Tabelle 7 angegeben.

**Tabelle 7:**

| Kalzium-Konzentration (mM) | % Erniedrigung Fluoreszenzintensität |
|---|---|
| 0 | 0 |
| 0,1 | 26,5 |
| 0,5 | 43,1 |
| 1,0 | 50,6 |
| 3,0 | 61,0 |
| 5,0 | 65,8 |
| 7,0 | 68,1 |
| 10,0 | 71,0 |

## Patentansprüche

1. Verbindungen der Formel I und II 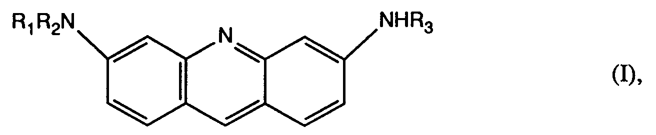 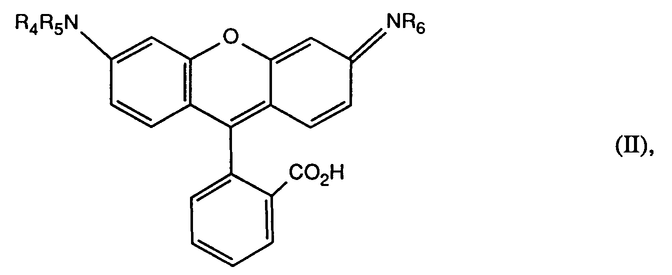 worin R₁ und R₃ sowie R₄ und R₆ für C₁-C₃₀-Alkyl oder C₁-C₃₀-Alkyl-CO- und R₂ sowie R₅ für H oder C₁-C₃₀-Alkyl stehen, mit der Massgabe, dass die Gesamtzahl der C-Atome der Alkylgruppen mindestens 12 beträgt, und deren Salze mit anorganischen oder organischen Säuren.

2. Verbindungen gemäss Anspruch 1, dadurch gekennzeichnet, dass R₂ für H steht.

3. Verbindungen gemäss Anspruch 1, dadurch gekennzeichnet, dass es sich bei den Alkylgruppen um lineare Alkylgruppen handelt.

4. Verbindungen gemäss Anspruch 1, dadurch gekennzeichnet, dass die Alkylgruppen 1 bis 22 C-Atome enthalten.

5. Verbindungen gemäss Anspruch 1, dadurch gekennzeichnet, dass R₁ und R₃ C₆-C₂₄-Alkyl oder C₆-C₂₄-Alkyl-CO- bedeuten und R₂ für H steht.

6. Verbindungen gemäss Anspruch 5, dadurch gekennzeichnet, dass R₁ und R₃ C₁₀-C₂₄-Alkyl oder C₁₀-C₂₄-Alkyl-CO- bedeuten.

7. Verbindungen gemäss Anspruch 5, dadurch gekennzeichnet, dass R₁ und R₃ C₁₄-C₂₂-Alkyl oder C₁₄-C₂₂-Alkyl-CO- bedeuten.

8. Verbindungen gemäss Anspruch 1, dadurch gekennzeichnet, dass R₅ H und R₄ und R₆ C₆-C₂₄-Alkyl darstellen.

9. Verbindungen gemäss Anspruch 8, dadurch gekennzeichnet, dass R₄ und R₆ C₁₀-C₂₄-Alkyl darstellen.

10. Verbindungen gemäss Anspruch 9, dadurch gekennzeichnet, dass R₄ und R₆ C₁₄-C₂₂-Alkyl darstellen.

11. Verbindungen gemäss Anspruch 1, dadurch gekennzeichnet, dass R₄ und R₅ C₁-C₆-Alkyl und R₆ C₁₀-C₂₄-Alkyl oder C₁₀-C₂₄-Alkyl-CO- bedeuten.

12. Verbindungen gemäss Anspruch 11, dadurch gekennzeichnet, dass R₄ und R₅ C₁-C₄-Alkyl und R₆ C₁₄-C₂₂-Alkyl oder C₁₄-C₂₂-Alkyl-CO- bedeuten.

13. Verbindungen gemäss Anspruch 12, dadurch gekennzeichnet, dass R₄ und R₅ Methyl oder Ethyl und R₆ C₁₆-C₂₂-Alkyl oder C₁₆-C₂₂-Alkyl-CO- bedeuten.

14. Verbindungen gemäss Anspruch 1, dadurch gekennzeichnet, dass sich die Salze der Verbindungen der Formeln I und II von HF, HCl, HBr, HI, H₂SO₃, H₂SO₄, H₃PO₃, H₃PO₄, HNO₂, HNO₃, HClO₄, HBF₄, HPF₆, HSbF₆, CF₃SO₃H, Toluolsulfonsäure, C₁-C₄-Alkyl- oder Phenylphosphonsäure, Ameisensäure, Essigsäure, Propionsäure, Benzoesäure, Mono- oder Di- oder Trichloressigsäure, Mono- oder Di- oder Trifluoressigsäure ableiten.

15. Verbindungen gemäss Anspruch 14, dadurch gekennzeichnet, dass sich die Salze der Verbindungen der Formeln I und II von HCl, HBr, H₂SO₄, HClO₄, HBF₄, HPF₆ und HSbF₆ ableiten.

16. Zusammensetzung aus
(a) einem transparenten Träger,
(b) der auf mindestens einer Seite mit einer transparenten Schicht beschichtet ist, die
(b1) ein hydrophobes Polymer
(b2) einen Weichmacher,
(b3) das Salz eines lipophilen Anions
(b4) ein Ionophor, der mit dem zu bestimmenden Ion einen Komplex bildet, und
(b5) eine Verbindung der Formel I oder II als Fluorophor enthält.

17. Zusammensetzung gemäss Anspruch 16, dadurch gekennzeichnet, dass die Verbindungen der Formeln I und II einen pKₐ-Wert von mindestens 8 aufweisen.

18. Zusammensetzung gemäss Anspruch 17, dadurch gekennzeichnet, dass der pKₐ-Wert mindestens 10 beträgt.

19. Zusammensetzung gemäss Anspruch 16, dadurch gekennzeichnet, dass es sich bei dem Träger um ein Glas handelt.

20. Zusammensetzung gemäss Anspruch 16, dadurch gekennzeichnet, dass die Dicke der Schicht auf dem Träger kann zum Beispiel von 0,01 bis 100 µm beträgt.

21. Zusammensetzung gemäss Anspruch 16, dadurch gekennzeichnet, dass das hydrophobe Polymer ein Molekulargewicht von mindestens 100 000 Dalton aufweist.

22. Zusammensetzung gemäss Anspruch 16, dadurch gekennzeichnet, dass das hydrophobe Polymer ein Homo- oder Copolymer aus Olefinen, Acrylaten, Methacrylaten, Vinylestern, Acrylnitril, Dienen, Styrol, Methylstyrol, Vinylchlorid, Vinylfluorid, Vinylidenchlorid, Vinylethern ist.

23. Zusammensetzung gemäss Anspruch 22, dadurch gekennzeichnet, dass es sich bei dem hydrophoben Polymer um Polyvinylchlorid, Polyvinylidenchlorid, Polyvinylfluorid, Polyacrylnitril, Polystyrol, Poly(methylstyrol), Polyacrylate und Polymethacrylate mit C₁-C₁₈-Alkylresten in den Estergruppen, Vinylchlorid/Vinylacetat-Copolymere, Vinylchlorid/Vinylacetat/Vinylalkohol-Copolymere, Vinylchlorid/Vinylacetat/Acrylnitril-Copoly mere, Vinylchlorid/Vinylidenchlorid-Copolymere, Vinylidenchlorid/Acrylnitril-Copolymere, Acrylnitril/Butadien/Styrol-Copolymere handelt.

24. Zusammensetzung gemäss Anspruch 22, dadurch gekennzeichnet, dass es sich bei dem hydrophoben Polymer um Homopolymere aus Vinylchlorid und Vinylidenchlorid sowie Copolymere aus Vinylchlorid und/oder Vinylidenchlorid und Acrylaten, Methacrylaten, Vinylestern, Vinylalkohol, Acrylnitril und Styrol handelt.

25. Zusammensetzung gemäss Anspruch 24, dadurch gekennzeichnet, dass es sich bei dem hydrophoben Polymer um Polyvinylchlorid handelt.

26. Zusammensetzung gemäss Anspruch 16, dadurch gekennzeichnet, dass der Weichmacher in einer Menge von 10 bis 90 Gew.-% enthalten ist, bezogen auf das Polymer.

27. Zusammensetzung gemäss Anspruch 16, dadurch gekennzeichnet, dass es sich bei dem Weichmacher um höhere Alkanole oder deren Ester, Ester von Fettsäuren mit Diolen oder Alkanolen, Ether mit höheren Alkanolen, Ester von Di- und Polycarbonsäuren und Ester von höheren Alkanolen und Phosphorsäure oder phosphoriger Säure handelt.

28. Zusammensetzung gemäss Anspruch 16, dadurch gekennzeichnet, dass als Salze mit lipophilen Anionen Alkalimetall-, Erdalkalimetall- und Ammoniumsalze mit gegebenenfalls substituierten Tetraphenylboraten enthalten sind.

29. Zusammensetzung gemäss Anspruch 28, dadurch gekennzeichnet, dass es sich bei den Kationen um Li^{⊕}, Na^{⊕}, K^{⊕}, Mg^{2⊕}, Ca^{2⊕}, NH₄^{⊕}, und die Ammoniumkationen von primären, sekundären und tertiären Aminen sowie quaternäre Ammoniumkationen handelt, die 1 bis 60 C-Atome enthalten.

30. Zusammensetzung gemäss Anspruch 28, dadurch gekennzeichnet, dass es sich bei den Boratanion um Tetraphenylborat handelt, dessen Phenylgruppen unsubstituiert oder mit ein oder mehreren C₁-C₄-Alkyl, C₁-C₄-Alkoxy, Halogen oder Trifluormethyl substituiert sind.

31. Zusammensetzung gemäss Anspruch 28, dadurch gekennzeichnet, dass es sich bei dem Boratanion um Natriumtetraphenylborat, Natriumtetra(3,5-bistrifluormethylphenyl)borat, Kaliumtetra(4-chlorphenyl)borat, Tetrabutylammonium-tetraphenylborat und Tetradodecyl(4-chlorphenyl)borat handelt.

32. Zusammensetzung gemäss Anspruch 16, dadurch gekennzeichnet, dass die Menge an Salzen mit lipophilen Anionen 0,01 bis 10 Gew.-% beträgt, bezogen auf die Menge an Polymer und Weichmacher.

33. Zusammensetzung gemäss Anspruch 16, dadurch gekennzeichnet, dass die Polymerschicht einen Ionophor in einer Menge von 0,01 bis 10 Gew.-% enthält, bezogen auf die Menge an Polymer und Weichmacher.

34. Zusammensetzung gemäss Anspruch 16, dadurch gekennzeichnet, dass als Ionophor Valinomycin enthalten ist, das selektiv Kaliumkationen zu binden vermag.

35. Zusammensetzung gemäss Anspruch 16, dadurch gekennzeichnet, dass die Menge an Verbindungen der Formeln I und II 0,01 bis 10 Gew.-%, beträgt, bezogen auf die Menge an Polymer und Weichmacher.

36. Zusammensetzung gemäss Anspruch 35, dadurch gekennzeichnet, dass die Menge an Verbindungen der Formeln I und II 0,1 bis 5 Gew.-% beträgt.

37. Zusammensetzung gemäss Anspruch 35, dadurch gekennzeichnet, dass die Menge an Verbindungen der Formeln I und II 0,1 bis 2 Gew.-% beträgt.

38. Optischer Sensor für die Bestimmung von Kationen in wässrigen Messproben insbesondere mittels Fluoroszenzspektrometrie, der aus (a) einem transparenten Träger besteht, (b) der auf mindestens einer Seite mit einer transparenten Schicht beschichtet ist, die
(b1) ein hydrophobes Polymer
(b2) einen Weichmacher,
(b3) das Salz eines lipophilen Anions
(b4) einen Ionophor, der mit dem zu bestimmenden Ion einen Komplex bildet, und
(b5) eine Verbindung der Formel I oder II als Fluorophor enthält.

39. Verfahren zur optischen Bestimmung von Kationen in wässrigen Messproben, bei dem man eine Zusammensetzung gemäss Anspruch 16 mit besagter wässriger Messprobe in Kontakt bringt und dann die Erniedrigung der Fluoreszenz in der aktiven Polymerschicht misst.

40. Verwendung der Verbindungen der Formeln I und II als Fluorophore zur optischen Bestimmung von Kationen in wässrigen Messproben.
